# EUROPÄISCHE PATENTSCHRIFT

(11) **EP 2 508 149 B1**
(45) Veröffentlichungstag und Bekanntmachung des Hinweises auf die Patenterteilung: **25.10.2017**
(21) Anmeldenummer: 12163070.1
(22) Anmeldetag: 04.04.2012
(51) Int. Cl.: A61F 2/38

(54) **Kniegelenkendoprothesensystem**
Knee joint prosthesis system
Système d'endoprothèse de genou

(30) Priorität: 06.04.2011 DE 102011001840
(43) Veröffentlichungstag der Anmeldung: 10.10.2012
(73) Patentinhaber: AESCULAP AG, 78532 Tuttlingen (DE)
(72) Erfinder: Hagen, Thomas, 78532 Tuttlingen (DE); Böttiger, Roland, 78604 Rietheim-Weilheim (DE); Maas, Allan, 78465 Konstanz (DE); Häfele, Claudia, 78603 Renquishausen (DE); Miehlke, Rolf, 65193 Wiesbaden (DE)
(74) Vertreter: Hoeger, Stellrecht & Partner Patentanwälte mbB

(56) Entgegenhaltungen:
- DE-A1- 10 012 059
- DE-A1- 19 915 053
- FR-A1- 2 876 276
- US-A- 5 782 925
- US-A1- 2002 107 576

## Beschreibung

Die vorliegende Erfindung betrifft ein Kniegelenkendoprothesensystem mit einem Tibiateil, einem am Tibiateil rotierbar lagerbaren Meniskusteil und einem mit dem Meniskusteil zusammenwirkenden Femurteil zur Ausbildung einer Kniegelenkendoprothese, wobei das System eine Rotationsbegrenzungseinrichtung zum Begrenzen einer Rotationsbewegung des Meniskusteils relativ zum Tibiateil umfasst und die Rotationsbegrenzungseinrichtung mindestens teilweise mit dem Tibiateil und/oder dem Meniskusteil lösbar verbindbar ist wobei ein zwischen dem Tibiateil und dem Meniskusteil ausgebildetes Drehlager vorgesehen ist, welches eine Rotationsachse senkrecht zu einer ebenen Tibiagleitfläche des Tibiateils definiert, an welcher Tibiagleitfläche eine ebene Meniskusgleitfläche des Meniskusteils anliegt, wobei das Kniegelenkendoprothesensystem eine Kopplungseinrichtung zum drehfesten Koppeln des Anschlagglieds mit dem Tibiateil umfasst und wobei die Kopplungseinrichtung ein erstes und ein zweites Kopplungsglied umfasst, welche in einer Kopplungsstellung kraft- und/oder formschlüssig in Eingriff stehen, und dass das erste Kopplungsglied am Anschlagglied und das zweite Kopplungsglied am Tibiateil angeordnet oder ausgebildet ist.

Kniegelenkendoprothesen dienen dem Ersatz natürlicher Kniegelenke, die aufgrund Abnutzung oder eines Traumas geschädigt sind. Mit den Kniegelenkendoprothesen wird nach Möglichkeit angestrebt, eine Bewegung des natürlichen Kniegelenks bestmöglich nachzubilden.

Kniegelenkendoprothesensysteme sind beispielsweise aus der DE 199 15 053 A1, der US 2002/0107576 A1, der FR 2 876 276 A1 und der DE 100 12 059 A1 bekannt. In der US 5,782,925 ist ein Probeimplantatsystem für eine Kniegelenkendoprothese offenbart.

Bei Kniegelenkendoprothesen der eingangs beschriebenen Art kann es passieren, dass das Meniskusteil relativ zum Tibiateil eine Rotation in eine nicht gewünschte Stellung ausführt. Dies kann insbesondere eine Verdrehung um 90° relativ zum Tibiateil sein.

Es ist daher eine Aufgabe der vorliegenden Erfindung, ein Kniegelenkendoprothesensystem der eingangs beschriebenen Art so zu verbessern, dass eine Rotation des Menikusteils relativ zum Tibiateil nur in einem beschränkten Umfang ermöglicht wird.

Diese Aufgabe wird bei einem Kniegelenkendoprothesensystem der eingangs beschriebenen Art erfindungsgemäß dadurch gelöst, dass das Anschlagglied in anteriorer Richtung weisend vom Drehlager absteht und dass ein zweiter Lagerbolzen vorgesehen ist, welcher wahlweise anstelle des ersten Lagerbolzens mit dem Tibiateil verbindbar ist.

Die erfindungsgemäß vorgeschlagene Weiterbildung hat den Vorteil, dass eine Rotation des Meniskusteils relativ zum Tibiateil in gewünschter Weise beschränkt werden kann. Ferner gestattet es die mindestens teilweise lösbare Verbindbarkeit der Rotationsbegrenzungseinrichtung mit dem Tibiateil oder dem Meniskusteil oder beiden Teilen, dass die Rotationsbegrenzungseinrichtung nur dann auch mit eine Rotationsbewegung des Meniskusteils und der Tibiateils relativ zueinander begrenzenden Elementen montiert wird, wenn dies tatsächlich erforderlich ist. Mit anderen Worten gestattet es die vorgeschlagene Rotationsbegrenzungseinrichtung, erst bei Bedarf angebracht oder durch Montieren mindestens eines Elements derselben am Meniskus- und/oder Tibiateil in ihrer Funktion zum Begrenzen einer Rotationsbewegung des Meniskusteils relativ zum Tibiateil aktiviert zu werden. Insgesamt lässt sich so ein modulares Kniegelenkendoprothesensystem ausbilden, welches wahlweise eine Rotationsbegrenzungseinrichtung vorsieht oder nicht beziehungsweise welches insbesondere auch noch nach einer Implantation eine Aktivierung der Rotationsbegrenzungseinrichtung oder Nachrüstung zum Begrenzen einer Rotationsbewegung des Meniskusteils relativ zum Tibiateil ermöglicht. Günstig ist es, dass zwischen dem Tibiateil und dem Meniskusteil ein Drehlager ausgebildet ist, welches eine Rotationsachse senkrecht zu einer ebenen Tibiagleitfläche des Tibiateils definiert, an welcher Tibiagleitfläche eine ebene Meniskusgleitfläche des Meniskusteils anliegt. Ein derart ausgebildetes Drehlager lässt sich auf einfache Weise herstellen und kann eine Rotationsbewegung des Meniskusteils und des Tibiateils relativ zueinander in definierter Weise vorgeben. Vorteilhaft ist es, dass das Anschlagglied in radialer Richtung bezogen auf die Rotationsachse vom Drehlager absteht. Ein derart angeordnetes beziehungsweise ausgebildetes Anschlagglied ermöglicht eine direkte Begrenzung einer Bewegung des Meniskusteils und des Tibiateils relativ zueinander. Das Anschlagglied kann wahlweise als Anschlagglied ausgebildet sein, welches eine Bewegung des Meniskusteils relativ zum Tibiateil begrenzt, oder entsprechend umgekehrt. Besonders kompakt ausbilden lässt sich das Kniegelenkendoprothesensystem, weil erfindungsgemäss das Anschlagglied in anteriorer Richtung weisend vom Drehlager absteht. Eine solche Anordnung ermöglicht es insbesondere, eine unerwünschte Wechselwirkung zwischen dem Anschlagglied und beispielweise am verbliebenen Femur noch erhaltener Bänder oder anderer Weichteile zu vermeiden. Eine solche Wechselwirkung könnte insbesondere dann auftreten, wenn das Anschlagglied in posteriorer Richtung weisend und über das Tibiateil hinaus abstehen würde. Erfindungsgemäss umfasst das Kniegelenkendoprothesensystem eine Kopplungseinrichtung zum drehfesten Koppeln des Anschlagglieds mit dem Tibiateil. Durch die Kopplungseinrichtung kann insbesondere sichergestellt werden, dass das Anschlagglied relativ zum Tibiateil nicht verdrehbar ist. Erfindungsgemäss umfasst die Kopplungseinrichtung ein erstes und ein zweites Kopplungsglied, welche in einer Kopplungsstellung kraft- und/oder formschlüssig in Eingriff stehen, wobei das erste Kopplungsglied am Anschlagglied und dass das zweite Kopplungsglied am Tibiateil angeordnet oder ausgebildet ist. Das Vorsehen insbesondere zueinander korrespondierender Kopplungsglieder am mindestens einen Anschlagglied und am Tibiateil ermöglicht auf einfache und sichere Weise eine direkte Kopplung der Teile aneinander. Erfindungsgemäss ist ein zweiter Lagerbolzen vorgesehen, welcher wahlweise anstelle des ersten Lagerbolzens mit dem Tibiateil verbindbar ist. Mit anderen Worten bedeutet dies, dass der erste Lagerbolzen bei Bedarf durch den zweiten Lagerbolzen ersetzt werden kann. Insbesondere können die Lagerbolzen unterschiedlich ausgestaltet sein, zum Beispiel speziell darauf abgestimmt sein, ob das Tibiateil einen Tibiaadapter umfasst oder nicht oder ob insbesondere ein Anschlagglied vorgesehen ist oder nicht.

Vorteilhaft ist es, wenn die Rotationsbegrenzungseinrichtung mindestens ein Anschlagglied umfasst, welches einen ersten und einen zweiten Anschlag definiert, welche in zueinander entgegengesetzte Rotationsrichtungen wirkend ausgebildet sind. Mindestens ein Anschlagglied vorzusehen, das heißt ein, zwei oder auch mehr Anschlagglieder, ermöglicht einen besonders einfachen Aufbau der Rotationsbegrenzungseinrichtung. Insbesondere wenn nur ein Anschlagglied vorgesehen ist, kann dieses gleichzeitig zwei Anschläge bilden, die in unterschiedliche Richtungen wirken.

Gemäß einer weiteren bevorzugten Ausführungsform der Erfindung kann vorgesehen sein, dass die Rotationsbegrenzungseinrichtung eine Rotationswinkelbereichvorgabeeinrichtung umfasst zum Definieren eines zulässigen Rotationswinkelbereichs für eine Rotation des Meniskusteils relativ zum Tibiateil. Mit der Rotationswinkelbereichvorgabeeinrichtung kann ein Rotationswinkel zwischen Meniskusteil und Tibiateil in gewünschter Weise vorgegeben werden. Beispielsweise können Rotationswinkel im Uhrzeigersinn und im Gegenuhrzeigersinn bezogen auf eine in anterior-posteriorer Richtung verlaufenden Ebene gleich oder auch unterschiedlich gewählt werden. Das heißt es ist beispielsweise denkbar, einen Rotationswinkel in medialer Richtung stärker zu begrenzen als in lateraler Richtung, oder umgekehrt.

Besonders einfach lässt sich ein Rotationswinkelbereich vorgeben, wenn die Rotationswinkelbereichvorgabeeinrichtung eine Aussparung umfasst, welche bezogen auf das Drehlager einen dem Rotationswinkelbereich entsprechenden Öffnungswinkel definiert. Die Aussparung kann beispielsweise am Tibiateil oder am Meniskusteil angeordnet oder ausgebildet sein.

Um einen Rotationswinkel auf einfache Weise zu begrenzen, ist es vorteilhaft, wenn das mindestens eine Anschlagglied mindestens teilweise in die Aussparung hineinragt. Beispielsweise können entweder eine Verdrehung des Anschlagglieds relativ zur Aussparung oder eine Verdrehung der Aussparung relativ zum Anschlagglied eine Bewegung des Meniskusteils und des Tibiateils relativ zueinander begrenzen, wenn beispielsweise das Abschlagglied teilweise an die Aussparung begrenzenden Flächen anschlägt.

Besonders einfach und kompakt ausbilden lässt sich die Rotationsbegrenzungseinrichtung, wenn die Rotationswinkelbereichvorgabeeinrichtung mit dem Anschlagglied zusammenwirkende Meniskusteilanschlagflächen am Meniskusteil umfasst. Beispielsweise kann das Abschlagglied relativ zum Tibiateil feststehend ausgebildet sein, so dass eine Rotation zwischen dem Meniskusteil und dem Tibiateil auf einfache Weise dadurch begrenzbar ist, dass bei einem maximal zulässigen Rotationswinkel die Meniskusteilanschlagflächen und das Anschlagglied aneinander anschlagen.

Besonders einfach wird der Aufbau einer Kniegelenkendoprothese, wenn die Meniskusteilanschlagflächen spiegelsymmetrisch zu einer die Rotationsachse enthaltenden, in anterior-posteriorer Richtung verlaufenden Spiegelebene ausgebildet sind. Beispielsweise bei einer symmetrischen Anordnung des Anschlagglieds können so Rotationswinkel im Uhrzeigersinn und im Gegenuhrzeigersinn in gleicher Weise begrenzt werden.

Auf besonders einfache Weise lässt sich die Rotationsbegrenzungseinrichtung ausbilden, wenn ein Abstand zwischen den Meniskusteilanschlagflächen in radialer Richtung bezogen auf die Rotationsachse zunimmt. Dies gestattet auf einfache Weise eine Verdrehung insbesondere des Meniskusteils relativ zum mindestens einen Anschlagglied.

Vorzugsweise definieren die Meniskusteilanschlagflächen Meniskusteilanschlagflächenebenen, welche sich in einer Schnittgeraden schneiden. Mit anderen Worten wird so insbesondere im Wesentlichen eine begrenzte Rotation in einem zwischen den Meniskusteilanschlagflächenebenen definierten Winkelbereich zwischen dem Meniskusteil und dem Tibiateil ermöglicht.

Besonders einfach und kompakt ausbilden lässt sich eine Kniegelenkendoprothese des Kniegelenkendoprothesensystems, wenn die Schnittgerade die Rotationsachse definiert.

Ferner kann es günstig sein, wenn die Meniskusteilanschlagflächen die Aussparung mindestens abschnittsweise begrenzen. So kann das mindestens eine Anschlagglied mit den Meniskusteilanschlagflächen auf einfache Weise zusammenwirken, wenn es in die Aussparung mindestens teilweise hineinragt.

Um die Kniegelenkendoprothese möglichst kompakt und mit wenigen Teilen ausbilden zu können, ist es vorteilhaft, wenn das Anschlagglied am Drehlager gehalten ist. Insbesondere kann es so auf einfache Weise in radialer Richtung von der Drehachse weg weisend ausgebildet werden.

Gemäß einer bevorzugten Ausführungsform der Erfindung kann vorgesehen sein, dass das Drehlager einen ersten, am Tibiateil festlegbaren Lagerbolzen mit einem rotationssymmetrischen Drehlagerabschnitt für das Meniskusteil umfasst. Beispielsweise kann so das Meniskusteil auf einfache Weise am Drehlagerabschnitt rotierbar gelagert werden. Vorzugsweise weist das Meniskusteil hierfür eine zum rotationssymmetrischen Drehlagerabschnitt korrespondierende Drehlagerabschnittaufnahme auf. Der rotationssymmetrische Drehlagerabschnitt kann insbesondere in Form eines oder mehrerer zylindrischer Abschnitte, beispielsweise auch in Form eines Ringflansches ausgebildet sein.

Um eine Revision der Kniegelenkendoprothese zu vereinfachen, ist es günstig, wenn der erste Lagerbolzen mit dem Tibiateil lösbar verbindbar ist. Insbesondere dann, wenn das mindestens eine Anschlagglied am Drehlager gehalten ist, kann so beispielsweise das Anschlagglied auch noch nach einer Implantation des Tibiateils und des Femurteils mit dem Drehlager gekoppelt oder gegebenenfalls von diesem entfernt werden.

Besonders einfach festlegen lässt sich der erste Lagerbolzen am Tibiateil, wenn er mit diesem verschraubbar ist.

Ein besonders einfacher und kompakter Aufbau der Kniegelenkendoprothese lässt sich erreichen, wenn das Anschlagglied am ersten Lagerbolzen gehalten oder mit diesem gekoppelt ist. Auf diese Weise kann das Anschlagglied bei Bedarf auch noch nachträglich am ersten Lagerbolzen montiert oder wieder von diesem entfernt werden. Vorzugsweise ist das Anschlagglied am ersten Lagerbolzen kraft- und/oder formschlüssig gehalten.

Um eine Montage des Anschlagglieds am ersten Lagerbolzen zu vereinfachen, ist es vorteilhaft, wenn das Anschlagglied eine Lagerbolzenaufnahme umfasst, in welche der erste Lagerbolzen mindestens teilweise eingreift. Beispielsweise kann die Lagerbolzenaufnahme in Form einer Durchbrechung ausgebildet sein, vorzugsweise in Form einer Bohrung. Beispielsweise kann das Anschlagglied einen Lagerring mit einer zentrischen Bohrung aufweisen, durch die der erste Lagerbolzen mindestens teilweise eingeführt werden kann. Der Lagerring kann sich bei entsprechender Anpassung seines Innendurchmessers an einen Außendurchmesser eines korrespondierenden zylindrischen Abschnitts des ersten Lagerbolzens ideal und unabhängig von einem Rotationswinkel am ersten Lagerbolzen abstützen.

Grundsätzlich wäre es denkbar, das mindestens eine Anschlagglied relativ zum Tibiateil bewegbar auszubilden. Um jedoch einen möglichst kompakten und stabilen Aufbau einer Kniegelenkendoprothese zu erreichen, ist es vorteilhaft, wenn das mindestens eine Anschlagglied relativ zum Tibiateil drehfest angeordnet ist. Eine derartige Ausgestaltung hat insbesondere den Vorteil, dass an einer Rotationslagerung zwischen dem Meniskusteil und dem Tibiateil grundsätzlich nichts geändert werden muss, um optional die Kniegelenkendoprothese mit dem mindestens einen Anschlagglied auszustatten.

Auf besonders einfache Weise kann eine drehfeste Kopplung zwischen dem mindestens einen Anschlagglied und dem Tibiateil erreicht werden, wenn die Kopplungseinrichtung ausgebildet ist zum kraft- und/oder formschlüssigen Koppeln des Anschlagglieds und des Tibiateils miteinander.

Besonders einfach herzustellen ist die Kopplungseinrichtung, wenn eines der Kopplungsglieder in Form einer Kopplungsaufnahme und eines in Form eines korrespondierenden Kopplungsvorsprungs ausgebildet ist. Dies gestattet es, die Kopplungsglieder mindestens formschlüssig, optional zusätzlich auch kraftschlüssig, miteinander zu koppeln.

Vorzugsweise ist die Kopplungsaufnahme am Tibiateil ausgebildet und in Richtung auf das Meniskusteil hin weisend geöffnet. Diese Ausgestaltung hat den Vorteil, dass die Tibiagleitfläche ohne Vorsprünge ausgebildet werden kann. Mit anderen Worten kann ein mit einer Kopplungsaufnahme versehenes Tibiateil auch genutzt werden, ohne dass das mindestens eine Anschlagglied an der Kniegelenkendoprothese angeordnet ist. Die Kopplungsaufnahme kann insbesondere in Form einer Vertiefung, beispielsweise in Form eines Sacklochs, ausgebildet sein.

Günstigerweise ist der Kopplungsvorsprung am Anschlagglied ausgebildet und steht in Richtung auf das Tibiateil hin weisend über die Meniskusgleitfläche vor. Ein derartiges Anschlagglied kann auf einfache Weise mit dem Tibiateil gekoppelt werden, wenn dieses eine zum Kopplungsvorsprung korrespondierende Kopplungsaufnahme aufweist.

Vorteilhaft ist es, wenn das Anschlagglied zwei in entgegengesetzte Richtungen weisende Anschlaggliedanschlagflächen für das Meniskusteil umfasst. Insbesondere kann so eine Kniegelenkendoprothese mit nur einem einzigen Anschlagglied ausgebildet werden.

Besonders einfach und kompakt ausbilden lässt sich eine Kniegelenkendoprothese, wenn die Anschlaggliedanschlagflächen parallel oder im Wesentlichen parallel zueinander verlaufen. Das Anschlagglied kann so beispielsweise teilweise stab- oder quaderförmig ausgebildet werden, wobei vorzugsweise Seitenflächen eines quaderförmigen oder stabförmigen Abschnitts des Anschlagglieds die Anschlaggliedanschlagflächen umfassen.

Ein besonders stabiler Aufbau einer Kniegelenkendoprothese kann erreicht werden, wenn das Tibiateil einstückig ausgebildet ist.

Gemäß einer weiteren bevorzugten Ausführungsform der Erfindung kann vorgesehen sein, dass das Tibiateil modular ausgebildet ist und eine Tibiaplatte und einen Tibiaadapter umfasst, dass die Tibiaplatte mit einem Schaft verbunden oder verbindbar ist und dass der Tibiaadapter mit der Tibiaplatte kraft- und/oder formschlüssig verbindbar ist. Eine derart modulares Tibiateil gestattet insbesondere eine individuelle Anpassung desselben an die Physiologie eines Patienten. Ferner hat der modulare Aufbau den Vorteil, dass das Tibiateil wahlweise mit oder ohne Tibiaadapter implantiert werden kann zur Ausbildung einer Kniegelenkendoprothese.

Vorzugsweise umfasst der Tibiaadapter die Tibiagleitfläche. Vorzugsweise umfassen sowohl die Tibiaplatte als auch der Tibiaadapter jeweils eine Tibiagleitfläche, die eine Anlagefläche für die Meniskusteilgleitfläche bilden. Dies ermöglicht es insbesondere, eine Kniegelenkendoprothese wahlweise mit oder ohne Tibiaadapter auszubilden.

Günstigerweise ist das Kopplungsglied des Tibiateils am Tibiaadapter angeordnet oder ausgebildet. Diese Ausgestaltung ermöglicht es insbesondere, beispielsweise ein bereits implantiertes Tibiateil, welches lediglich eine Tibiaplatte mit einer Tibiagleitfläche aufweist, optional mit einem Tibiaadapter auszustatten, welcher mit dem mindestens einen Anschlagglied wahlweise koppelbar ist. Eine solche Ausgestaltung ermöglicht insbesondere die Nachrüstung bereits implantierter, herkömmlicher Tibiateile oder herkömmlicher Kniegelenkendoprothesen mit einer Rotationsbegrenzungseinrichtung. Wenn beispielsweise die Tibiaplatte kein Kopplungsglied zum Koppeln mit einem Anschlagglied aufweist, kann der Tibiaadapter beispielsweise auf die Tibiaplatte aufgesetzt werden, welcher dann aufgrund des an ihm ausgebildeten Kopplungsglieds eine Kopplung mit dem mindestens einen Anschlagglied ermöglicht, um so eine Begrenzung einer Rotation des Meniskusteils relativ zum Tibiateil zu erreichen. Mit anderen Worten kann also das Kniegelenkendoprothesensystem so beispielsweise mit einem Nachrüstsatz umfassend einen Tibiaadapter und eine Rotationsbegrenzungseinrichtung bereitgestellt werden, wobei die Rotationsbegrenzungseinrichtung insbesondere einen entsprechenden Lagerbolzen und mindestens ein Anschlagglied umfassen kann. Der Nachrüstsatz kann ferner insbesondere auch ein mit dem mindestens einen Anschlagglied zusammenwirkendes Meniskusteil umfassen, um das Meniskusteil der bereits implantierten, in ihrer Rotation einzuschränkenden Kniegelenkendoprothese zu ersetzen.

Vorteilhafterweise sichert das Drehlager den Tibiaadapter gegen ein Lösen von der Tibiaplatte. Insbesondere kann so beispielsweise ein Lösen des Tibiaadapters vom Tibiateil in einer Richtung parallel zur Rotationsachse verhindert werden. Ein Lösen in einer Ebene quer zur Rotationsachse kann beispielsweise verhindert werden durch eine entsprechende formschlüssige Ausbildung des Tibiaadapters einerseits und der Tibiaplatte andererseits.

Günstigerweise ist der Tibiaadapter an der Tibiaplatte bezogen auf die Rotationsachse drehfest festgelegt. Damit kann eine im Wesentlichen starr verbundene Einheit zwischen der Tibiaplatte und dem Tibiaadapter ausgebildet werden zur Ausbildung des Tibiateils.

Günstig ist es, wenn das Drehlager einen zweiten, am Tibiateil festlegbaren Lagerbolzen mit einem rotationssymmetrischen Drehlagerabschnitt für das Meniskusteil umfasst und wenn der zweite Lagerbolzen wahlweise anstatt des ersten Lagerbolzens zur Ausbildung der Kniegelenkendoprothese am Tibiateil montierbar ist. Je nachdem, ob das Tibiateil einen Tibiaadapter umfasst oder nicht, kann beispielsweise entweder der erste oder der zweite Lagerbolzen am Tibiateil montiert werden. Ferner können die beiden Lagerbolzen auch so ausgebildet sein, dass der eine beispielsweise eine Montage eines Anschlagglieds gestattet, der andere jedoch nicht.

Besonders vorteilhaft ist es, wenn der zweite Lagerbolzen nur ohne das Anschlagglied am Tibiateil montierbar ist. Beispielsweise kann dann der zweite Lagerbolzen, der eine Montage eines Anschlagglieds nicht gestattet, weil er nicht hierfür ausgebildet ist, vom Tibiateil entfernt und stattdessen der erste Lagerbolzen mit einem Anschlagglied montiert werden, um eine Rotation des Meniskusteils relativ zum Tibiateil zu begrenzen.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Darstellung einer implantierten Kniegelenkendoprothese mit Rotationsbegrenzungseinrichtung;
- Figur 2:: eine perspektivische, teilweise durchbrochene Explosionsdarstellung der Kniegelenkendoprothese aus Figur 1;
- Figur 3:: eine Schnittansicht längs Linie 3-3 in Figur 2;
- Figur 4A:: eine teilweise durchbrochene Draufsicht auf die in Figur 3 dargestellte Anordnung in einer nicht ausgelenkten Grundstellung;
- Figur 4B:: eine Ansicht analog Figur 4A, jedoch in einer ausgelenkten Stellung des Meniskusteils relativ zum Tibiateil; und
- Figur 5:: eine Explosionsdarstellung eines alternativen Ausführungsbeispiels einer Kniegelenkendoprothese.

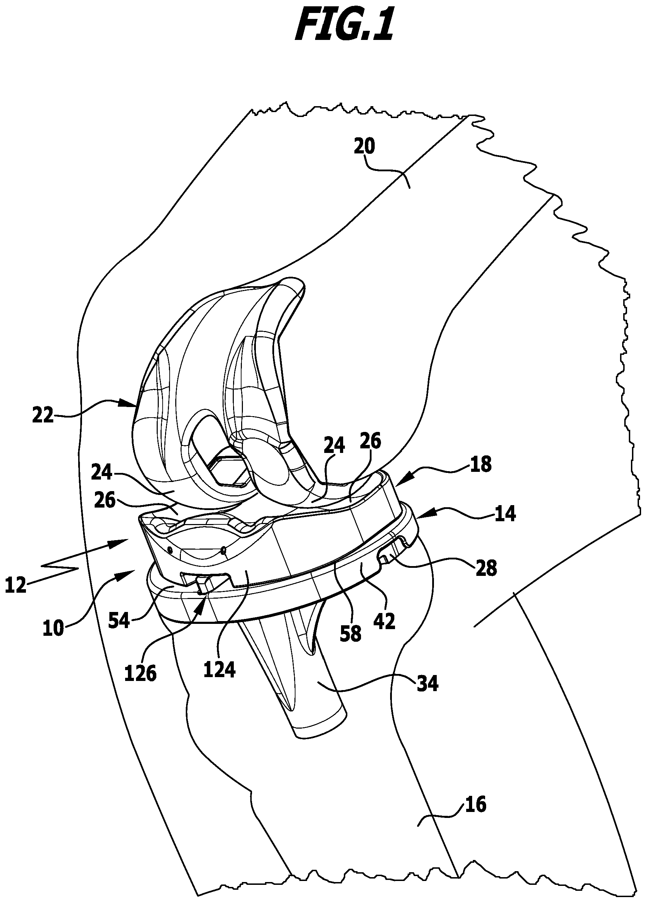

In Figur 1 ist schematisch ein erstes Ausführungsbeispiel einer Kniegelenkendoprothese 10 eines insgesamt mit dem Bezugszeichen 12 bezeichneten Kniegelenkendoprothesensystems dargestellt. Es umfasst einen Tibiateil 14 zum Implantieren an einer Tibia 16, ein am Tibiateil rotierbar gelagertes Meniskusteil 18 sowie ein mit dem Meniskusteil 18 zusammenwirkendes und an einem Femur 20 implantierbares Femurteil 22.

Das Femurteil weist zwei künstliche Kondylenflächen 24 auf, die an in Richtung auf das Femurteil 22 hin weisenden Gelenkflächen 26 des Meniskusteils anliegen und an diesen abrollen und/oder abgleiten können.

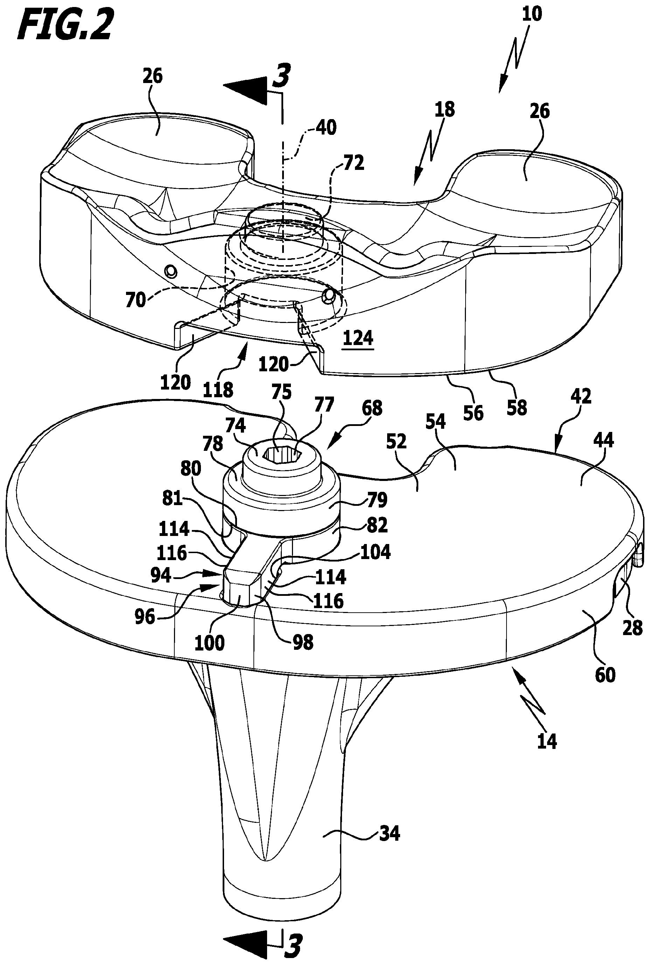

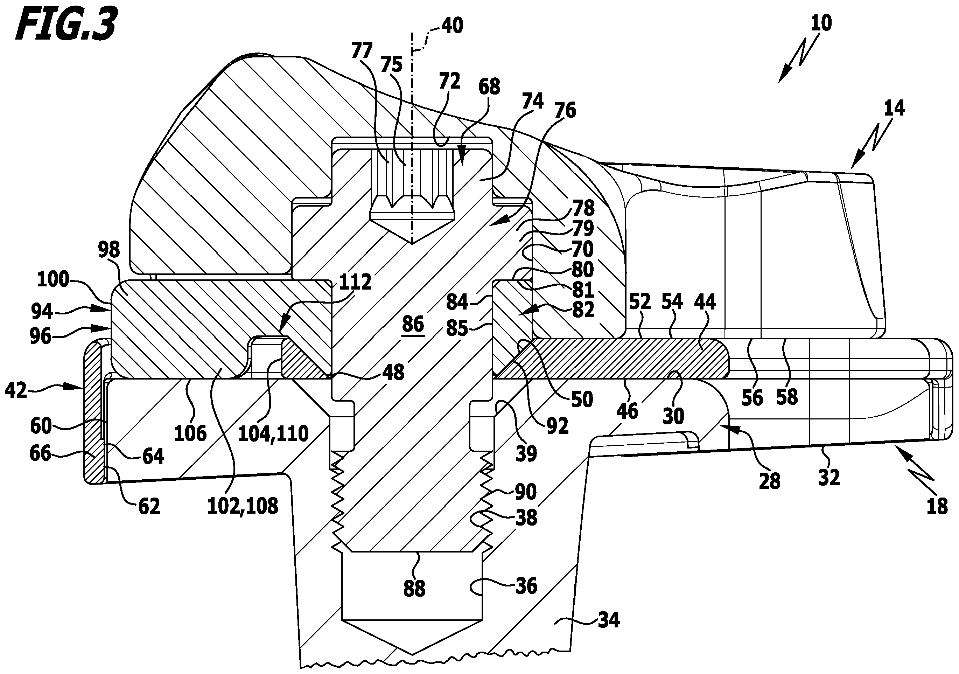

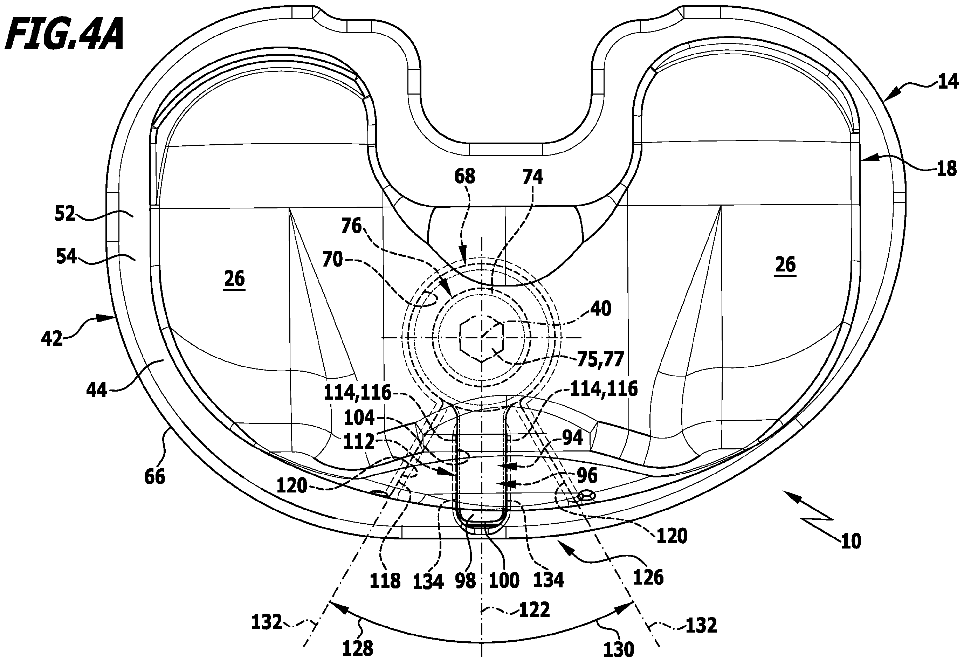

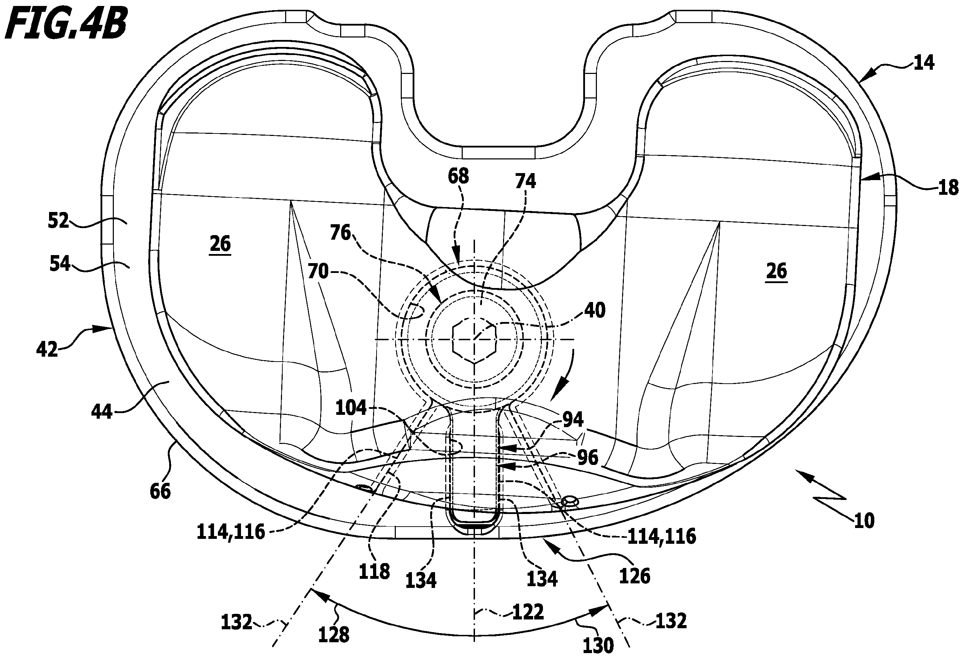

Das Tibiateil 14 ist bei dem in den Figuren 1 bis 4B dargestellten Ausführungsbeispiel modular aufgebaut. Es umfasst eine Tibiaplatte 28, welche in einer Draufsicht im Wesentlichen nierenförmig ausgebildet ist und eine ebene und in Richtung auf das Meniskusteil 18 hin weisende erste Tibiagleitfläche 30 definiert. Von einer Unterseite 32 der Tibiaplatte 28 steht im Wesentlichen senkrecht ein kurzer Schaftadapter 34 ab, welcher mit nicht näher in den Figuren dargestellten Schäften kraft- und/oder formschlüssig in Eingriff gebracht werden kann. Insbesondere ist bei dem in den Figuren 1 bis 4B dargestellten Ausführungsbeispiel vorgesehen, einen Schaft gewünschter Länge mit dem Schaftadapter 34 zu verschrauben. Selbstverständlich kann statt des Schaftadapters 34 auch jede andere Form von Adapter vorgesehen sein, um, falls dies gewünscht ist, die Tibiaplatte 28 mit einem Schaft zu koppeln. Grundsätzlich wäre es auch denkbar, eine Tibiaplatte ohne Schaftadapter vorzusehen, und zwar unabhängig von einer Ausgestaltung der Kniegelenkendoprothesensystem im Übrigen umfassten Teile.

In die Tibiaplatte 28 ist ein Sackloch 36 eingebracht, welches sich noch ein kurzes Stück in den Schaftadapter 34 hinein erstreckt. Ein kurzer, zylindrischer Abschnitt des Sacklochs 36 ist mit einem Innengewinde 38 versehen. Eine Längsachse des Sacklochs 36 definiert eine Rotationsachse 40 für eine Rotation des Meniskusteils 18 relativ zum Tibiateil 14. Ferner weist das Sackloch 36 eine von der ersten Tibiagleitfläche 30 weg weisende konische Erweiterung 39 auf, welche eine Ringfläche definiert, die bezogen auf eine Längsachse des Sacklochs 36 um etwa 45° geneigt ist.

Die erste Tibiagleitfläche 30 ist praktisch vollständig überdeckt von einem Tibiaadapter 42, welcher in einer Draufsicht eine zur Tibiaplatte 28 im Wesentlichen ähnliche Form aufweist. Er umfasst eine ebene Adapterplatte 44, deren Unterseite 46 eben ist, eine Anlagefläche definiert und flächig an der ersten Tibiagleitfläche 30 anliegt.

Die Adapterplatte 44 weist eine kreisförmige Durchbrechung auf, deren Innendurchmesser einem Innendurchmesser des Sacklochs 36 entspricht. Die Durchbrechung 48 ist ausgehend von der Unterseite 46 im Innendurchmesser erweitert und bildet eine konisch geformte zweite Erweiterung 50, die in Richtung auf das Meniskusteil 18 hin gerichtet ist. Ein Neigungswinkel der durch die Erweiterung 50 ausgebildeten Ringfläche entspricht dem zwischen der Erweiterung 39 und der Rotationsachse 40.

Eine Oberseite 52 der Adapterplatte 44 des insgesamt einstückig ausgebildeten Tibiaadapters 42 bildet eine zweite Tibiagleitfläche 54. Sie ist ebenfalls eben und dient als Anlagefläche für das Meniskusteil 18, welches mit seiner Unterseite 56, die ebenfalls eben ausgebildet ist und eine Meniskusgleitfläche 58 definiert, flächig an der zweiten Tibiagleitfläche 54 anliegt. Von der Adapterplatte 44 steht ein teilweise umlaufender Rand 60 senkrecht ab und begrenzt so quasi in einer Richtung quer zur Rotationsachse 40 eine Tibiaplattenaufnahme 62 zum Aufnehmen der Tibiaplatte 28. Der Rand 60 liegt mit einer in Richtung auf die Rotationsachse 40 hin weisenden Innenseite 64 an einer in radialer Richtung von der Rotationsachse 40 weg weisenden Außenseite 66 der Tibiaplatte 28 an. Der Rand 60 ermöglicht somit eine im Wesentlichen formschlüssige Verbindung zwischen der Tibiaplatte 28 und dem Tibiaadapter 42, wodurch der Tibiaadapter 42 an der Tibiaplatte 28 gegen eine Bewegung in einer Richtung quer zur Rotationsachse 40 gesichert ist.

Um das Meniskusteil 18 am Tibiateil 14 rotierbar zu lagern, ist ein Drehlager 68 vorgesehen. Dieses umfasst eine hohlzylindrische Ausnehmung 70, die ausgehend von der Unterseite 56 in das Meniskusteil 18 eingearbeitet ist. Ausgehend von einem in Richtung auf das Tibiateil 14 hin weisenden Boden 72 der Ausnehmung 70, erweitert sich die Ausnehmung 70 einstufig im Innendurchmesser nach etwa einem Drittel ihrer Länge parallel zur Rotationsachse 40. In die Ausnehmung 70 ragt ein Kopf 74 eines ersten Lagerbolzens 76 des Drehlagers 68 hinein. Der Kopf 74 umfasst einen in radialer Richtung abstehenden, zylindrischen Ringflansch 78, welcher eine in Richtung auf die zweite Tibiagleitfläche 54 hin weisende und parallel zu dieser verlaufende Ringfläche 81 definiert. Der Ringflansch 78 bildet einen rotationssymmetrischen Drehlagerabschnitt 79 für das Meniskusteil 14. An der Ringfläche 81 liegt eine korrespondierende Ringfläche 80 eines Lagerrings 82 an. Der Lagerring 82 weist eine zur Rotationsachse 40 koaxiale Bohrung 84 auf, deren Innendurchmesser einem Außendurchmesser eines Schafts 86 des ersten Lagerbolzens 76 entspricht. Die Bohrung 84 bildet eine Lagerbolzenaufnahme 85, welche der erste Lagerbolzen 76 durchsetzt, also mindestens teilweise in diese eingreift.

Ausgehend von einem freien Ende 88, welches dem Kopf 74 gegenüberliegt, ist der erste Lagerbolzen 76 auf etwa einem Viertel seiner Länge mit einem Außengewinde 90 versehen, welches korrespondierend zum Innengewinde 38 ausgebildet ist, so dass der erste Lagerbolzen 76 mit der Tibiaplatte 28 verschraubbar und damit lösbar verbindbar ist. Am Kopf 74 ist eine sacklochartige Werkzeugelementaufnahme 75 in Form eines Innensechskant 77 ausgebildet. Die Werkzeugelementaufnahme 75 ist koaxial zur Rotationsachse 40 ausgebildet und weist in einer Richtung von der Tibiaplatte 28 weg. Sie ermöglicht mit einem korrespondierenden Einschraubwerkzeug auf einfache Weise das Einschrauben des ersten Lagerbolzens in das mit dem Innengewinde 38 versehenes Sackloch an der Tibiaplatte 28.

Der Lagerring 82 ist mit einer Fase versehen, welche flächig an der durch die Erweiterung 50 definierten Ringfläche anliegt. Mit dem ersten Lagerbolzen 76, welcher in das Sackloch 36 hineingeschraubt ist, kann somit der Tibiaadapter 42 klemmend zwischen dem an der Ringfläche 81 anliegenden Lagerring 82 und der ersten Tibiagleitfläche 30 klemmend gehalten werden.

Der Lagerring 82 bildet einen Teil eines Anschlagglieds 94, welches wiederum einen Teil einer insgesamt mit dem Bezugszeichen 96 bezeichneten Rotationsbegrenzungseinrichtung bildet. Vom Lagerring 82 steht in anteriorer Richtung weisend ein quaderförmiger Vorsprung 98 ab, von welchem von einem in anteriorer Richtung weisenden Ende 100 ein Kopplungsvorsprung 102 in Richtung auf die erste Tibiagleitfläche 30 hin weisend absteht. Der Kopplungsvorsprung 102 greift in eine korrespondierend ausgebildete Kopplungsaufnahme 104 am Tibiaadapter 42 ein und liegt mit seiner Unterseite 106 auf der ersten Tibiagleitfläche 30 auf. Durch die beschriebene Montage des ersten Lagerbolzens am Tibiateil 14 und die miteinander zusammenwirkenden Kopplungsglieder 108 und 110 einer insgesamt mit dem Bezugszeichen 112 bezeichneten Kopplungseinrichtung zum kraft- und/oder formschlüssigen Koppeln des Anschlagglieds 94 und des Tibiateils 14 miteinander ist das Anschlagglied 94 mit dem Tibiateil 14 drehfest gekoppelt.

Am Vorsprung 98 definieren zwei parallel zueinander verlaufende und voneinander weg weisende Seitenflächen 114 jeweils einen Anschlag 116, welche in zueinander entgegengesetzte Rotationsrichtungen wirkend ausgebildet sind. Die Anschläge 116 definieren voneinander weg weisende Anschlaggliedanschlagflächen 134 für das Meniskusteil.

Das Anschlagglied 94 der Rotationsbegrenzungseinrichtung 96 wirkt direkt mit eine Aussparung 118 seitlich begrenzenden Meniskusteilanschlagflächen 120 zusammen. Die Meniskusteilanschlagflächen 120 sind spiegelsymmetrisch zu einer die Rotationsachse 40 enthaltenden, in anterior-posteriorer Richtung verlaufenden Spiegelebene 122 ausgebildet. Die Aussparung 118 ist am Meniskusteil 18 ausgehend von dessen Unterseite 56 ausgebildet und erstreckt sich ausgehend von der Bohrung 84 in anteriorer Richtung bis zu einer Vorderseite 124 des Meniskusteils 18. Durch die besondere Ausbildung der Meniskusteilanschlagflächen 120 nimmt ein Abstand zwischen diesen in radialer Richtung bezogen auf die Rotationsachse 40 zu. Man kann auch sagen, die Aussparung 118 wird mit zunehmendem Abstand von der Rotationsachse 40 breiter. Des Weiteren definieren die Meniskusteilanschlagflächen 120 Meniskusteilanschlagflächenebenen 132, welche sich in einer Schnittgeraden schneiden, bei der es sich um die Rotationsachse 40 handelt.

Das Anschlagglied 94 ragt teilweise, nämlich mit seinem Vorsprung 98, in die Aussparung 118 hinein. Die Aussparung 118 bildet einen Teil einer Rotationswinkelbereichvorgabeeinrichtung 126 zum Definieren eines zulässigen Rotationswinkelbereichs 130 für eine Rotation des Meniskusteils 18 relativ zum Tibiateil 14. Der Rotationswinkelbereich 130 wird vorgegeben durch einen zwischen den Meniskusteilanschlagflächen 120 definierten Öffnungswinkel 128, welcher bei dem in den Figuren 1 bis 4B dargestellten Ausführungsbeispiel etwa 60° beträgt.

Die Funktionsweise der Kniegelenkendoprothese 10 wird nachfolgend erläutert.

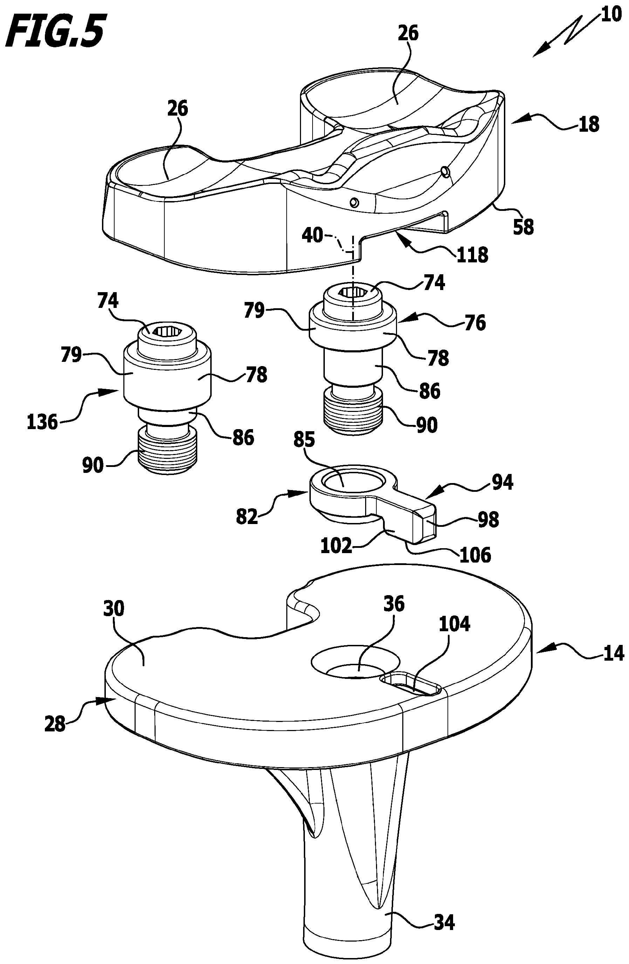

Durch den modularen Aufbau des Kniegelenkendoprothesensystems 12 ist es möglich, zunächst nur die Tibiaplatte 28, gegebenenfalls mit einem an dieser montierten Schaft, an der Tibia 16 zu verankern. Des Weiteren kann das Femurteil 22 am Femur 20 in bekannter Weise verankert werden. In einem nächsten Schritt kann dann ein zweiter Lagerbolzen 136, der schematisch in Figur 5 dargestellt ist, mit der Tibiaplatte 28 verschraubt werden. Der zweite Lagerbolzen 136 ist prinzipiell identisch ausgebildet wie der erste Lagerbolzen 76, jedoch ist er etwas kürzer als dieser, und zwar verkürzt um eine Höhe des Anschlagglieds 94, so dass der Ringflansch 78 direkt an der ersten Tibiagleitfläche 30 anliegen kann.

Im nächsten Schritt wird das oben beschriebene Meniskusteil 18 auf die Tibiaplatte 28 derart aufgesetzt, dass der Kopf des zweiten Lagerbolzens in die Ausnehmung 70 eingreift. Das Meniskusteil 18 ist dann frei rotierbar relativ zum Tibiateil 14 um die Rotationsachse 40.

Falls ein Operateur eine Rotation des Tibiateils 14 relativ zum Meniskusteil 18 beschränken möchte, wird zunächst das Meniskusteil 18 wieder herausgenommen und anschließend der zweite Lagerbolzen 136 wieder herausgeschraubt. In einem nächsten Schritt wird der Tibiaadapter 42 auf die Tibiaplatte 28 aufgesetzt. Der erste Lagerbolzen 76 wird durch die Lagerbolzenaufnahme 85 des Anschlagglieds 94 hindurchgesteckt und mit der Tibiaplatte 28 verschraubt. Anschließend kann das Meniskusteil 18 wieder aufgesetzt werden. Das Anschlagglied 94 ist in der oben beschriebenen Weise durch die Kopplungseinrichtung 112 gegen eine Rotation relativ zum Tibiaadapter 42 um die Rotationsachse 40 gesichert. Das Meniskusteil 18 kann um die Rotationsachse 40 nur so weit verdreht werden, bis eine der Meniskusteilanschlagflächen 120 an einer der Anschlaggliedanschlagflächen 134 anschlägt. Auf diese Weise kann eine unerwünschte Rotationsstellung des Meniskusteils 18 relativ zum Tibiateil 14 verhindert werden.

Der oben beschriebene Tibiaadapter 42 ermöglicht die Nachrüstung einer Tibiaplatte ohne Kopplungsglied 110 mit einer Rotationsbegrenzungseinrichtung 96. Damit ist es möglich, bereits implantierte Kniegelenkendoprothese noch nachträglich mit einer Rotationsbegrenzungseinrichtung 96 auszustatten. Hierfür ist es lediglich erforderlich, das ursprünglich implantierte Meniskusteil, wenn dieses keine Aussparung 118 in der oben beschriebenen Form aufweist, gegen das oben beschriebene Meniskusteil 18 auszuwechseln, den ursprünglichen zweiten Lagerbolzen 136 zu entfernen und gegen den oben beschriebenen ersten Lagerbolzen 76 mit daran gekoppeltem Anschlagglied 94 auszuwechseln. Das Anschlagglied 94 in Verbindung mit dem Tibiaadapter 42 gestattet dann nachträglich eine Begrenzung einer Rotationsbewegung des Meniskusteils 18 relativ zum Tibiateil 14.

Eine weitere Variante einer Kniegelenkendoprothese 10 des Kniegelenkendoprothesensystems 12 ist in Figur 5 schematisch dargestellt. Sie unterscheidet sich von der oben beschriebenen Kniegelenkendoprothese 10 dadurch, dass die Kopplungsaufnahme 104 nicht am Tibiaadapter 42, sondern in der Tibiaplatte 28 ausgebildet ist. Dies ermöglicht es, dass der Kopplungsvorsprung 102 am Anschlagglied 94 direkt in die Kopplungsaufnahme 104 an der Tibiaplatte 28 eingreifen kann, um eine drehfeste Verbindung zwischen dem Anschlagglied 94 und dem lediglich die Tibiaplatte 28 umfassenden Tibiateil 14 herzustellen.

Nach der Implantation des Tibiateils 14 und des nicht dargestellten Femurteils 20 kann zunächst nicht der erste Lagerbolzen 76, sondern der zweite Lagerbolzen 136 in das Sackloch 36 eingeschraubt werden. Der zweite Lagerbolzen 136 bei dieser Variante unterscheidet sich vom ersten Lagerbolzen 76 dadurch, dass er bei identischer Gesamtlänge einen etwa doppelt so hohen Ringflansch 78 aufweist, dessen Höhe in der Summe der Höhe des Ringflanschs 78 des ersten Lagerbolzens 76 und der Höhe des Lagerrings 82 entspricht. Mit der Ausnehmung 70 bildet er somit zusammen ein Drehlager 68 für das Meniskusteil 18 aus.

Falls wider Erwarten während oder auch nach Implantation der Kniegelenkendoprothese 10 eine Rotationsbegrenzung gewünscht wird, kann das Meniskusteil 18 zunächst entfernt werden. Der zweite Lagerbolzen 136 wird dann durch den ersten, mit dem Anschlagglied 94 gekoppelten Lagerbolzen 76 ausgewechselt und dann der erste Lagerbolzen 76 wieder mit der Tibiaplatte 28 verschraubt. Anschließend kann das Meniskusteil 18 wieder eingesetzt werden. Die so ausgebildete Kniegelenkendoprothese 10 entspricht in ihrer Funktion der in den Figuren 1 bis 4B dargestellten Kniegelenkendoprothese 10, wobei sie jedoch keinen Tibiaadapter 42 aufweist.

## Patentansprüche

1. Kniegelenkendoprothesensystem (12) mit einem Tibiateil (14), einem am Tibiateil (14) rotierbar lagerbaren Meniskusteil (18) und einem mit dem Meniskusteil (18) zusammenwirkenden Femurteil (22) zur Ausbildung einer Kniegelenkendoprothese (10), wobei das System eine Rotationsbegrenzungseinrichtung (96) zum Begrenzen einer Rotationsbewegung des Meniskusteils (18) relativ zum Tibiateil (14) umfasst und die Rotationsbegrenzungseinrichtung (96) mindestens teilweise mit dem Tibiateil (14) und/oder dem Meniskusteil (18) lösbar verbindbar ist, wobei ein zwischen dem Tibiateil (14) und dem Meniskusteil (18) ausgebildetes Drehlager (68) mit einem ersten Lagerbolzen (76) vorgesehen ist, welches eine Rotationsachse (40) senkrecht zu einer ebenen Tibiagleitfläche (54) des Tibiateils (14) definiert, an welcher Tibiagleitfläche (54) eine ebene Meniskusgleitfläche (58) des Meniskusteils (18) anliegt, wobei das Kniegelenkendoprothesensystem eine Kopplungseinrichtung (112) zum drehfesten Koppeln eines Anschlagglieds (94) mit dem Tibiateil (14) umfasst und wobei die Kopplungseinrichtung (112) ein erstes und ein zweites Kopplungsglied (108, 110) umfasst, welche in einer Kopplungsstellung kraft- und/oder formschlüssig in Eingriff stehen, und wobei das erste Kopplungsglied (108) am Anschlagglied (94) und das zweite Kopplungsglied (110) am Tibiateil (14) angeordnet oder ausgebildet ist, **dadurch gekennzeichnet, dass** das Anschlagglied (94) in anteriorer Richtung weisend vom Drehlager (68) absteht und dass ein zweiter Lagerbolzen (136) vorgesehen ist, welcher wahlweise anstelle des ersten Lagerbolzens (76) mit dem Tibiateil (14) verbindbar ist.

2. Kniegelenkendoprothesensystem nach Anspruch 1, **dadurch gekennzeichnet, dass** die Rotationsbegrenzungseinrichtung (96) mindestens ein Anschlagglied (94) umfasst, welches einen ersten und einen zweiten Anschlag (116) definiert, welche in zueinander entgegengesetzte Rotationsrichtungen wirkend ausgebildet sind.

3. Kniegelenkendoprothesensystem nach einem der voranstehenden Ansprüche, **dadurch gekennzeichnet, dass** die Rotationsbegrenzungseinrichtung (96) eine Rotationswinkelbereichvorgabeeinrichtung (126) umfasst zum Definieren eines zulässigen Rotationswinkelbereichs (130) für eine Rotation des Meniskusteils (18) relativ zum Tibiateil (14).

4. Kniegelenkendoprothesensystem nach Anspruch 3, **dadurch gekennzeichnet, dass** die Rotationswinkelbereichvorgabeeinrichtung (126) eine Aussparung (118) umfasst, welche bezogen auf das Drehlager (68) einen dem Rotationswinkelbereich (130) entsprechenden Öffnungswinkel (128) definiert.

5. Kniegelenkendoprothesensystem Anspruch 4, **dadurch gekennzeichnet, dass** das mindestens eine Anschlagglied (94) mindestens teilweise in die Aussparung (118) hineinragt.

6. Kniegelenkendoprothesensystem nach einem der voranstehenden Ansprüche, **dadurch gekennzeichnet, dass** die Rotationswinkelbereichvorgabeeinrichtung (126) mit dem Anschlagglied (94) zusammenwirkende Meniskusteilanschlagflächen (120) am Meniskusteil (18) umfasst und dass die Meniskusteilanschlagflächen (120) Meniskusteilanschlagflächenebenen (132) definieren, welche sich in einer Schnittgeraden schneiden.

7. Kniegelenkendoprothesensystem nach einem der voranstehenden Ansprüche, **dadurch gekennzeichnet, dass** das Anschlagglied (94) am Drehlager (68) gehalten ist.

8. Kniegelenkendoprothesensystem nach einem der voranstehenden Ansprüche, **dadurch gekennzeichnet, dass** das Drehlager (68) einen ersten, am Tibiateil (14) festlegbaren Lagerbolzen (76) mit einem rotationssymmetrischen Drehlagerabschnitt (79) für das Meniskusteil (18) umfasst.

9. Kniegelenkendoprothesensystem nach Anspruch 8, **dadurch gekennzeichnet, dass** das Anschlagglied (94) eine Lagerbolzenaufnahme (85) umfasst, in welche der erste Lagerbolzen (76) mindestens teilweise eingreift.

10. Kniegelenkendoprothesensystem nach einem der voranstehenden Ansprüche, **dadurch gekennzeichnet, dass** eines der Kopplungsglieder (108, 110) in Form einer Kopplungsaufnahme (104) und eines in Form eines korrespondierenden Kopplungsvorsprungs (102) ausgebildet ist.

11. Kniegelenkendoprothesensystem nach Anspruch 10, **dadurch gekennzeichnet, dass** die Kopplungsaufnahme (104) am Tibiateil (14) ausgebildet ist und in Richtung auf das Meniskusteil (18) hin weisend geöffnet ist und/oder dass der Kopplungsvorsprung (102) am Anschlagglied (94) ausgebildet ist und in Richtung auf das Tibiateil (14) hin weisend über die Meniskusgleitfläche (58) vorsteht.

12. Kniegelenkendoprothesensystem nach einem der voranstehenden Ansprüche, **dadurch gekennzeichnet, dass** das Anschlagglied (94) zwei in entgegengesetzte Richtungen weisende Anschlaggliedanschlagflächen (134) für das Meniskusteil (18) umfasst.

13. Kniegelenkendoprothesensystem nach einem der voranstehenden Ansprüche, **dadurch gekennzeichnet, dass** das Tibiateil (14) modular ausgebildet ist und eine Tibiaplatte (28) und einen Tibiaadapter (42) umfasst, dass die Tibiaplatte (28) mit einem Schaft verbunden oder verbindbar ist und dass der Tibiaadapter (42) mit der Tibiaplatte (28) kraft- und/oder formschlüssig verbindbar ist.

14. Kniegelenkendoprothesensystem nach einem der voranstehenden Ansprüche, **dadurch gekennzeichnet, dass** das Drehlager (68) einen zweiten, am Tibiateil (14) festlegbaren Lagerbolzen(136) mit einem rotationssymmetrischen Drehlagerabschnitt (79) für das Meniskusteil (18) umfasst und dass der zweite Lagerbolzen (136) wahlweise anstatt des ersten Lagerbolzens (76) zur Ausbildung der Kniegelenkendoprothese (10) am Tibiateil (14) montierbar ist.

15. Kniegelenkendoprothesensystem nach Anspruch 14, **dadurch gekennzeichnet, dass** der zweite Lagerbolzen (136) nur ohne das Anschlagglied (94) am Tibiateil (14) montierbar ist.

## Claims

1. Knee joint endoprosthesis system (12) with a tibia part (14), a meniscus part (18) rotatably mountable on the tibia part (14) and a femur part (22) interacting with the meniscus part (18) for formation of a knee joint endoprosthesis (10), the system comprising a rotation limiting device (96) for limiting a rotational movement of the meniscus part (18) relative to the tibia part (14), and the rotation limiting device (96) being at least partially releasably connectable to the tibia part (14) and/or to the meniscus part (18), a rotary bearing (68) formed between the tibia part (14) and the meniscus part (18) being provided with a first bearing bolt (76) which defines an axis of rotation (40) perpendicular to a flat tibia slide surface (54) of the tibia part (14), against which tibia slide surface (54) a flat meniscus slide surface (58) of the meniscus part (18) lies, the knee joint endoprosthesis system comprising a coupling device (112) for rotationally fixedly coupling a stop member (94) to the tibia part (14), and the coupling device (112) comprising a first and a second coupling member (108, 110) which engage with force locking and/or positive locking in a coupled position, and the first coupling member (108) being arranged or formed on the stop member (94) and the second coupling member (110) on the tibia part (14), **characterized in that** the stop member (94), facing in the anterior direction, protrudes from the rotary bearing (68), and **in that** a second bearing bolt (136) is provided, which is optionally connectable instead of the first bearing bolt (76) to the tibia part (14).

2. Knee joint endoprosthesis system in accordance with claim 1, **characterized in that** the rotation limiting device (96) comprises a least one stop member (94) which defines a first and a second stop (116) which are formed so as to act in directions of rotation opposite to each other.

3. Knee joint endoprosthesis system in accordance with any one of the preceding claims, **characterized in that** the rotation limiting device (96) comprises a rotation angle range prescribing device (126) for defining an allowable rotation angle range (130) for a rotation of the meniscus part (18) relative to the tibia part (14).

4. Knee joint endoprosthesis system in accordance with claim 3, **characterized in that** the rotation angle range prescribing device (126) comprises a recess (118) which in relation to the rotary bearing (68) defines an opening angle (128) corresponding to the rotation angle range (130).

5. Knee joint endoprosthesis system in accordance with claim 4, **characterized in that** the at least one stop member (94) extends at least partially into the recess (118).

6. Knee joint endoprosthesis system in accordance with any one of the preceding claims, **characterized in that** the rotation angle range prescribing device (126) comprises meniscus part stop surfaces (120) on the meniscus part (18), which interact with the stop member (94), and **in that** the meniscus part stop surfaces (120) define meniscus part stop surface planes (132) which intersect in a line of intersection.

7. Knee joint endoprosthesis system in accordance with any one of the preceding claims, **characterized in that** the stop member (94) is held on the rotary bearing (68).

8. Knee joint endoprosthesis system in accordance with any one of the preceding claims, **characterized in that** the rotary bearing (68) comprises a first bearing bolt (76) fixable on the tibia part (14) with a rotationally symmetrical rotary bearing portion (79) for the meniscus part (18).

9. Knee joint endoprosthesis system in accordance with claim 8, **characterized in that** the stop member (94) comprises a bearing bolt receptacle (85) in which the first bearing bolt (76) engages at least partially.

10. Knee joint endoprosthesis system in accordance with any one of the preceding claims, **characterized in that** one of the coupling members (108, 110) is constructed in the form of a coupling receptacle (104) and one in the form of a corresponding coupling projection (102).

11. Knee joint endoprosthesis system in accordance with claim 10, **characterized in that** the coupling receptacle (104) is formed on the tibia part (14) and is open in the direction facing the meniscus part (18) and/or **in that** the coupling projection (102) is formed on the stop member (94) and, facing in the direction towards the tibia part (14), projects over the meniscus slide surface (58).

12. Knee joint endoprosthesis system in accordance with any one of the preceding claims, **characterized in that** the stop member (94) comprises two stop member stop surfaces (134) facing in opposite directions for the meniscus part (18).

13. Knee joint endoprosthesis system in accordance with any one of the preceding claims, **characterized in that** the tibia part (14) is of modular construction and comprises a tibia plate (28) and a tibia adapter (42), **in that** the tibia plate (28) is connected or connectable to a shaft, and **in that** the tibia adapter (42) is connectable with force locking and/or positive locking to the tibia plate (28).

14. Knee joint endoprosthesis system in accordance with any one of the preceding claims, **characterized in that** the rotary bearing (68) comprises a second bearing bolt (136) fixable to the tibia part (14) with a rotationally symmetrical rotary bearing portion (79) for the meniscus part (18), and **in that** the second bearing bolt (136) is optionally mountable instead of the first bearing bolt (76) on the tibia part (14) for formation of the knee joint endoprosthesis (10).

15. Knee joint endoprosthesis system in accordance with claim 14, **characterized in that** the second bearing bolt (136) is only mountable on the tibia part (14) without the stop member (94).

## Revendications

1. Système d'endoprothèse d'articulation du genou (12) comprenant une pièce de tibia (14), une pièce de ménisque (18) pouvant être montée de manière rotative sur la pièce de tibia (14), et une pièce de fémur (22) interagissant avec la pièce de ménisque (18) pour former une endoprothèse d'articulation du genou (10), système dans lequel il est prévu un dispositif de limitation de rotation (96) destiné à limiter un mouvement de rotation de la pièce de ménisque (18) par rapport à la pièce de tibia (14), et le dispositif de limitation de rotation (96) peut être relié de manière démontable, au moins partiellement, avec la pièce de tibia (14) et/ou la pièce de ménisque (18),
dans lequel un palier de rotation (68) formé entre la pièce de tibia (14) et la pièce de ménisque (18) est prévu avec un premier tourillon de palier (76) qui définit un axe de rotation (40) perpendiculaire à une surface de glissement de tibia (54) plane de la pièce de tibia (14), surface de glissement de tibia (54) sur laquelle s'appuie une surface de glissement de ménisque (58) plane de la pièce de ménisque (18),
le système d'endoprothèse d'articulation du genou comprenant un dispositif de couplage (112) pour assurer le couplage fixe en rotation d'un organe de butée (94) avec la pièce de tibia (14),
et le dispositif de couplage (112) comprenant un premier et un deuxième organe de couplage (108, 110), qui, dans une position de couplage, sont en prise par adhérence et/ou par complémentarité de formes, et le premier organe de couplage (108) étant agencé ou formé sur l'organe de butée (94) et le deuxième organe de couplage (110) sur la pièce de tibia (14),
**caractérisé en ce que** l'organe de butée (94) fait saillie du palier de rotation (68) en étant dirigé en direction antérieure, et **en ce qu'**il est prévu un deuxième tourillon de palier (136), qui peut être relié sélectivement à la pièce de tibia (14), à la place du premier tourillon de palier (76).

2. Système d'endoprothèse d'articulation du genou selon la revendication 1, **caractérisé en ce que** le dispositif de limitation de rotation (96) comprend au moins un organe de butée (94), qui définit une première et une deuxième butée (116), réalisées de façon à être actives dans des sens de rotation mutuellement opposés.

3. Système d'endoprothèse d'articulation du genou selon l'une des revendications précédentes, **caractérisé en ce que** le dispositif de limitation de rotation (96) comporte un dispositif de prescription de plage d'angle de rotation (126) destiné à définir une plage d'angle de rotation admissible (130) pour une rotation de la pièce de ménisque (18) par rapport à la pièce de tibia (14).

4. Système d'endoprothèse d'articulation du genou selon la revendication 3, **caractérisé en ce que** le dispositif de prescription de plage d'angle de rotation (126) comporte un évidement (118), qui, par rapport au palier de rotation (68), définit un angle d'ouverture (128) correspondant à la plage d'angle de rotation (130).

5. Système d'endoprothèse d'articulation du genou selon la revendication 4, **caractérisé en ce que** ledit au moins un organe de butée (94) s'engage au moins partiellement dans l'évidement (118).

6. Système d'endoprothèse d'articulation du genou selon l'une des revendications précédentes, **caractérisé en ce que** le dispositif de prescription de plage d'angle de rotation (126) comprend des surfaces de butée de pièce de ménisque (120) sur la pièce de ménisque (18), qui interagissent avec l'organe de butée (94), et **en ce que** le surfaces de butée de pièce de ménisque (120) définissent des plans de surface de butée de pièce de ménisque (132), qui se coupent selon une droite d'intersection.

7. Système d'endoprothèse d'articulation du genou selon l'une des revendications précédentes, **caractérisé en ce que** l'organe de butée (94) est maintenu sur le palier de rotation (68).

8. Système d'endoprothèse d'articulation du genou selon l'une des revendications précédentes, **caractérisé en ce que** le palier de rotation (68) comprend un premier tourillon de palier (76) pouvant être fixé à la pièce de tibia (14) et présentant un tronçon de palier de rotation (79) à symétrie de rotation pour la pièce de ménisque (18).

9. Système d'endoprothèse d'articulation du genou selon la revendication 8, **caractérisé en ce que** l'organe de butée (94) comporte un logement d'accueil de tourillon de palier (85) dans lequel s'engage au moins partiellement le premier tourillon de palier (76).

10. Système d'endoprothèse d'articulation du genou selon l'une des revendications précédentes, **caractérisé en ce que** l'un des organes de couplage (108, 110) est réalisé sous la forme d'un logement de couplage (104) et l'un sous la forme d'une protubérance de couplage (102) correspondante.

11. Système d'endoprothèse d'articulation du genou selon la revendication 10, **caractérisé en ce que** le logement de couplage (104) est formé sur la pièce de tibia (14) et est ouvert en direction de la pièce de ménisque (18), et/ou **en ce que** la protubérance de couplage (102) est formée sur l'organe de butée (94) et fait saillie de la surface de glissement de ménisque (58) en direction de la pièce de tibia (14).

12. Système d'endoprothèse d'articulation du genou selon l'une des revendications précédentes, **caractérisé en ce que** l'organe de butée (94) comprend deux surfaces de butée d'organe de butée (134) pour la pièce de ménisque (18), qui sont dirigées vers des directions opposées.

13. Système d'endoprothèse d'articulation du genou selon l'une des revendications précédentes, **caractérisé en ce que** la pièce de tibia (14) est d'une configuration modulaire et comprend un plateau tibial (28) et un adaptateur de tibia (42), **en ce que** le plateau tibial (28) est relié ou peut être relié à un corps allongé, et **en ce que** l'adaptateur de tibia (42) peut être relié par adhérence et/ou complémentarité de formes au plateau tibial (28).

14. Système d'endoprothèse d'articulation du genou selon l'une des revendications précédentes, **caractérisé en ce que** le palier de rotation (68) comprend un deuxième tourillon de palier (136) pouvant être fixé à la pièce de tibia (14) et présentant un tronçon de palier de rotation (79) à symétrie de rotation pour la pièce de ménisque (18), et **en ce que** le deuxième tourillon de palier (136) peut être monté sélectivement sur la pièce de tibia (14), à la place du premier tourillon de palier (76), pour former l'endoprothèse d'articulation du genou (10).

15. Système d'endoprothèse d'articulation du genou selon la revendication 14, **caractérisé en ce que** le deuxième tourillon de palier (136) ne peut être monté que sans l'organe de butée (94) sur la pièce de tibia (14).
